Europäisches Patentamt

European Patent Office

Office européen des brevets

(11) Veröffentlichungsnummer: **0 397 643**
**A2**

(12) **EUROPÄISCHE PATENTANMELDUNG**

(21) Anmeldenummer: 90890135.8

(51) Int. Cl.5: **A61F 5/48**

(22) Anmeldetag: **07.05.90**

(30) Priorität: **11.05.89 AT 1126/89**

(43) Veröffentlichungstag der Anmeldung:
**14.11.90 Patentblatt 90/46**

(84) Benannte Vertragsstaaten:
**AT BE CH DE ES FR GB IT LI NL SE**

(71) Anmelder: **Hainy, Johann**

**A-4173 ST. Veit 135(AT)**

(72) Erfinder: **Hainy, Johann**

**A-4173 ST. Veit 135(AT)**

(74) Vertreter: **Hübscher, Helmut, Dipl.-Ing. et al
Patentanwälte Dipl.-Ing. Gerhard Hübscher
Dipl.-Ing. Helmut Hübscher Dipl.-Ing. Heiner
Hübscher Spittelwiese 7
A-4020 Linz(AT)**

(54) **Feuchtigkeitswächter, insbesondere für Windeln u. dgl.**

(57) Ein Feuchtigkeitswächter (1) besteht aus einem eine Befestigungseinrichtung aufweisenden Gehäuse (2), das einen batteriegespeisten, über einen elektronischen Schalter (8) einschaltbaren Signalgeber (7) und zwei auswärts ragende, an den Schalter (8) angeschlossene Elektroden aufnimmt, wobei der Schalter (8) durch eine äußere feuchtigkeitsbedingte Leitungsverbindung der Elektroden im Einschaltsinn betätigbar ist und die Befestigungseinrichtung zwei voneinander elektrisch isolierte Halterungen (4) umfaßt, die an den Schalter (8) angeschlossen sind.

Um einen einfachen, leicht zu handhabenden und stets einsatzbereiten Feuchtigkeitswächter zu erreichen, bilden die Elektroden die Halterungen (4).

FIG.1

EP 0 397 643 A2

Die Erfindung bezieht sich auf einen Feuchtigkeitswächter, insbesondere für Windeln u. dgl., mit einem eine Befestigungseinrichtung aufweisenden Gehäuse, das einen batteriegespeisten, über einen elektronischen Schalter einschaltbaren Signalgeber und zwei auswärts ragende, an den Schalter angeschlossene Elektroden aufnimmt, wobei der Schalter durch eine äußere feuchtigkeitsbedingte Leitungsverbindung der Elektroden im Einschaltsinn betätigbar ist und die Befestigungseinrichtung zwei voneinander elektrisch isolierte Halterungen umfaßt, die an den Schalter angeschlossen sind.

Wie die DE-OSen 29 48 768 und 27 27 930 zeigen, gibt es bereits Feuchtigkeitswächter als Warngeräte für Bettnässer oder zur Füllstandsanzeige von Behältern u. dgl., wobei das eigentliche Gerät mit Gehäuse, Batterie, optischem oder akustischem Signalgeber und der Signalschaltung für sich auf geeignete Weise an einer Schutzhose, einer Matte, einer Behälterwand u. dgl. zu befestigen ist und die aus dem Gerätegehäuse herausgeführten Elektroden über Druckknopfkontakte od. dgl. Leitungsverbindungen an speziellen Feuchtigkeitsfühlern angeschlossen werden müssen. Gegebenenfalls frei vorragende Elektroden lassen sich dabei höchstens für Füllstands-Anzeige- und Warngeräte verwenden. Gemäß der DE-OS 36 08 403 wurde zwar auch schon ein Feuchtigkeits wächter für Wegwerfwindeln vorgeschlagen, der über Druckknöpfe als Halterungen an in die Windeln eingearbeitete Elektroden fixiert wird, doch ist hier jede Windel mit eigenen Elektroden auszustatten und die Störmöglichkeit der Druckknopfkontakte in Kauf zu nehmen. Die bekannten Feuchtigkeitswächter verlangen daher alle einen entsprechenden Aufwand und sind darüber hinaus recht umständlich zu handhaben, so daß sie kaum als einfache, mühelos ansetzbare und funktionssichere Feuchtigkeitswächter für Babywindeln u. dgl. dienen können.

Der Erfindung liegt daher die Aufgabe zugrunde, diese Mängel zu beseitigen und einen Feuchtigkeitswächter der eingangs geschilderten Art zu schaffen, der sich durch seinen einfachen, handlichen Aufbau auszeichnet und mit wenigen Handgriffen schnell und geschickt und ohne jede Verletzungsge fahr auch bei Babywindeln angebracht werden kann.

Die Erfindung löst diese Aufgabe dadurch, daß die Elektroden die Halterungen bilden. Durch die zusätzliche Nutzung der Elektroden als Befestigungseinrichtung entsteht ein aufwandsarmes, rationelles Gerät, das ein separates Installieren von Feuchtigkeitsfühlern u. dgl. überflüssig macht und bereits durch das Befestigen des Gehäuses funktionsgerecht angeordnet ist. Es gibt keine in einer Windeleinlage od. dgl. verbleibenden Teile mehr, es igt nicht auf ordnungsgemäße Kontakte zwischen Elektroden und Fühlern zu achten und der leicht zu handhabende Feuchtigkeitswächter läßt sich mit einem Griff an beliebiger Stelle anbringen und ist sofort funktionstüchtig.

Als Halterungen for die Befestigungseinrichtung können zweckmäßigerweise Klemmen od. dgl. verwendet werden, doch besonders günstig ist es, wenn erfindungsgemäß Sicherheits oder Anstecknadeln als Halterungen vorgesehen sind, da diese Nadeln gut in das feucht werdende Material, beispielsweise Babywindeln, eingeführt werden können und auch feuchtigkeitsundurchlässige Schutzfolien od. dgl. von Windelhosen u. dgl. einfach durchstechen.

Ist das Gehäuse knopfförmig ausgestaltet, ergibt sich ein zusätzlicher Schmuckeffekt, die runden Knopfformen verhindern Verletzungen auch bei Kleinkindern und nicht zuletzt wird die Handhabung der Feuchtigkeitswächter durch die Knopfform beim Anstecken erleichtert.

In der Zeichnung ist der Erfindungsgegenstand rein schematisch anhand eines Ausführungsbeispieles veranschaulicht, und zwar zeigen

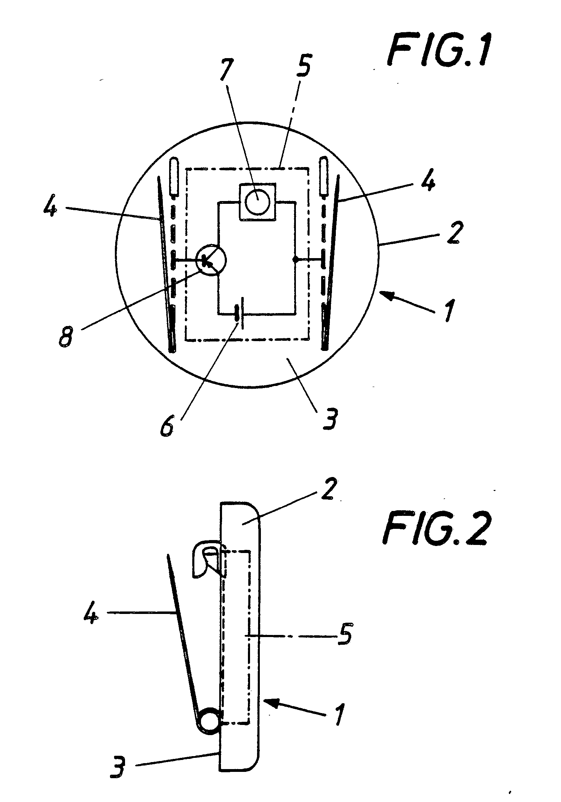

Fig. 1 einen erfindungsgemäßen Feuchtigkeitswächter in Untersicht und

Fig. 2 in Seitenansicht.

Der dargestellte Feuchtigkeitswächter 1 besteht aus einem knopfförmigen Gehäuse 2, an dessen Unterseite 3 zwei voneinander elektrisch isolierte Anstecknadeln 4 als Befestigungseinrichtung vorgesehen sind. Das Gehäuse 2 nimmt in seinem Inneren eine Signalschaltung 5 auf, die einen über eine Batterie 6 gespeisten, akustischen Signalgeber 7 und einen Transistor 8 als elektronischen Schalter zum Einschalten des Signalgebers 7 umfaßt. Die Anstecknadeln 4, die an die Signalschaltung 5 angeschlossen sind, dienen als Elektroden for die Betätigung des Transistors 8, so daß durch eine feuchtigkeitsbedingte Leitungsverbindung der beiden Anstecknadeln 4 der Transistor 8 durchschaltet und der Signalgeber 7 durch sein akustisches Signal das Vorhandensein von Feuchtigkeit anzeigt.

Durch die Ausbildung der Elektroden als Anstecknadeln 4 od. dgl. Halterungen für das Gehäuse 2 ergibt sich ein besonders handlicher, jederzeit und überall einsatzfähiger und funktionstüchtiger Feuchtigkeitswächter, der sich speziell far Babywindeln eignet. Dabei spielt es keine Rolle, welche Art von Signalschaltung oder Warnsignal, beispielsweise eine Liedmelodie, verwendet wird, da das Wesentliche in der funktionellen Einheit von Halterungen und Elektroden zu sehen ist.

## Ansprüche

1. Feuchtigkeitswächter (1), insbesondere für Windeln u. dgl., mit einem eine Befestigungseinrichtung aufweisenden Gehäuse (2), das einen batteriegespeisten, über einen elektronischen Schalter
(8) einschaltbaren Signalgeber (7) und zwei auswärts ragende, an den Schalter (8) angeschlossene
Elektroden aufnimmt, wobei der Schalter (8) durch
eine äußere feuchtigkeitsbedingte Leitungsverbindung der Elektroden im Einschaltsinn betätigbar ist
und die Befestigungseinrichtung zwei voneinander
elektrisch isolierte Halterungen (4) umfaßt, die an
den Schalter (8) angeschlossen sind, dadurch gekennzeichnet, daß die Elektroden die Halterungen
(4) bilden.

2. Feuchtigkeitswächter nach Anspruch 1, dadurch gekennzeichnet, daß Sicherheitsnadeln (4)
oder Anstecknadeln als Halterungen vorgesehen
sind.

3. Feuchtigkeitswächter nach Anspruch 1 oder
2, dadurch gekennzeichnet, daß das Gehäuse (2)
knopfförmig ausgestaltet ist.

FIG.1

FIG.2